Europäisches Patentamt

European Patent Office

Office européen des brevets

(19)

(11) Numéro de publication: **0 356 335 B1**

(12) **FASCICULE DE BREVET EUROPEEN**

(45) Date de publication de fascicule du brevet: **10.05.95**

(51) Int. Cl.[6]: **C12N 15/67**, C12N 1/19, C12P 21/02

(21) Numéro de dépôt: **89402328.2**

(22) Date de dépôt: **23.08.89**

(54) **Peptide signal et séquences d'ADN codant pour celui-ci.**

(30) Priorité: **24.08.88 FR 8811188**

(43) Date de publication de la demande:
**28.02.90 Bulletin 90/09**

(45) Mention de la délivrance du brevet:
**10.05.95 Bulletin 95/19**

(84) Etats contractants désignés:
**AT BE CH DE ES FR GB GR IT LI LU NL SE**

(56) Documents cités:
EP-A- 0 170 266
EP-A- 0 177 343

NUCLEIC ACIDS RESEARCH, vol. 12, no. 13, juillet 1984, pages 5145-5164, IRLPress Limited, Oxford, GB; M.E.E. WATSON: "Compilation of published signalsequences"

EUR. J. BIOCHEM., vol. 133, 1983, pages 17-21, FEBS; G. VON HEIJNE:

(73) Titulaire: **SANOFI**
**32-34, rue Marbeuf**
**F-75008 Paris (FR)**

(72) Inventeur: **Legoux, Richard**
**En Manart**
**Le Faget**
**F-31460 Caraman (FR)**
Inventeur: **Leplatois, Pascal**
**en Saint Pierre**
**Cambon Les Lavaur**
**I-81470 Cuq Toulza (FR)**
Inventeur: **Joseph-Liauzun, Evelyne**
**8, rue du Palais**
**F-31650 Saint Orens De Gameville (FR)**
Inventeur: **Loison, Gérard**
**15, rue François Ricardie**
**F-31300 Toulouse (FR)**
Inventeur: **Roskam, Willem**
**Décédé**
**/ (FR)**

(74) Mandataire: **Gillard, Marie-Louise et al**
**Cabinet Beau de Loménie**
**158, rue de l'Université**
**F-75340 Paris Cédex 07 (FR)**

THE JOURNAL OF BIOLOGICAL CHEMISTRY, vol. 262, no. 13, 5 mai 1987, pages 6328-6333, The American Society of Biological Chemists, Inc., Baltimore, MD., US; L.T. DUONG et al.: "Synthetic signal peptide and analogs display differentactivities in mammalian and plant in vitro secretion systems"

GENE, vol. 39, 1985, pages 247-254, Elsevier Science Publishers; G.L. GRAY etal.: "Periplasmic production of correctly processed human growth hormone inEscherichia coli: natural and bacterial signal sequences are interchangeable"

**Description**

La présente invention concerne un nouveau peptide-signal, les séquences d'ADN codant pour celui-ci, les vecteurs d'expression portant l'une de ces dernières, les bactéries gram-négatives transformées par ceux-ci ainsi qu'un procédé de production périplasmique d'un polypeptide à l'aide de ces dernières.

Il est connu que les bactéries gram-négatives produisent naturellement des polypeptides, synthétisés sous la forme d'un précurseur dans le cytoplasme et exportés vers le périplasme, espace compris entre la membrane cytoplasmique et la paroi bactérienne, où ils s'accumulent sous la forme d'un polypeptide mature, c'est-à-dire d'un polypeptide capable d'assurer son action biologique spécifique. Parmi ces polypeptides figurent notamment des enzymes, telles que par exemple la phosphatase alcaline.

Il est connu également que l'on peut utiliser des bactéries gram négatives pour leur faire produire dans leur périplasme un polypeptide qui leur est étranger. Une telle production périplasmique revêt un intérêt certain car la séparation dudit polypeptide des autres constituants du périplasme est plus facile que la séparation de celui-ci des autres composants du cytoplasme, telle qu'elle est requise dans le cas d'une production avec accumulation cytoplasmique. Elle est également intéressante car le polypeptide s'accumule sous sa forme mature sans qu'il y ait addition d'une méthionine N-terminale qu'il faudrait ensuite éliminer et sans qu'il y ait adoption d'une conformation secondaire défavorable.

On sait que, pour que sa production soit périplasmique, un polypeptide doit être synthétisé sous la forme d'un précurseur correspondant au polypeptide mature allongé à son extrémité N-terminale d'un peptide constitué généralement de 15 à 30 acides aminés appelé peptide-signal. Ce peptide-signal, qui joue un rôle déterminant dans la sécrétion du polypeptide, est clivé au cours du processus libérant ainsi le polypeptide mature dans le périplasme.

S'agissant d'adapter des bactéries à la production périplasmique d'un polypeptide qui leur était étranger et notamment un polypeptide d'origine eucaryote, les premiers travaux ont consisté à transformer les bactéries à l'aide d'un vecteur d'expression portant une séquence d'ADN codant pour un précurseur naturel dudit polypeptide. Cette stratégie s'est à plusieurs reprises avérée peu adaptée en terme de quantité à une production industrielle.

Une tentative pour apporter une solution satisfaisante a consisté à remplacer le peptide-signal naturel du polypeptide par celui d'un polypeptide bactérien synthétisé sous la forme d'un précurseur. La demande de brevet EP-A-0177343 présente des exemples de mise en oeuvre de tels peptides-signaux.

La demanderesse, constatant que le choix d'un peptide-signal bactérien pouvait déterminer pour le précurseur d'un polypeptide hétérologue, (notamment d'origine eucaryote) au fur et à mesure de sa synthèse dans la bactérie, la prise par celui-ci d'une conformation secondaire inappropriée, a conçu un nouveau peptide-signal qui permet un bon rendement de production périplasmique de polypeptides hétérologues biologiquement actifs.

L'invention concerne donc un nouveau peptide-signal de formule :

M X K S T L L L L F L L L C L P S W N A G A

où

A, C, F, G, K, L, M, N, P, S, T et W désignent des acides aminés selon le code suivant :

| | |
|---|---|
| A = Alanine | M = Méthionine |
| C = Cystéine | N = Asparagine |
| F = Phénylalanine | P = Proline |
| G = Glycine | S = Sérine |
| K = Lysine | T = Thréonine |
| L = Leucine | W = Tryptophane |

et X représente une liaison directe entre M et K, un acide aminé choisi dans l'ensemble des 20 acides aminés du code génétique ou un peptide comportant 2, 3 ou 4 acides aminés choisis chacun indépendamment l'un de l'autre dans l'ensemble des 20 acides aminés du code génétique.

Un peptide-signal particulièrement apprécié est celui dans lequel X représente une liaison directe entre M et K, ou tout ou partie du peptide de séquence APSG.

L'invention concerne, selon un autre aspect, les séquences d'ADN codant pour le peptide-signal selon l'invention. Toutes les séquences que permet la dégénérescence du code génétique peuvent être utilisées. Deux séquences particulièrement appréciées sont les suivantes :

```
5'    ATG-GCT-CCA-TCT-GGC-AAA-TCC-ACG-CTG

      CTT-CTC-TTA-TTT-CTG-CTC-CTG-TGC-CTG

      CCC-TCT-TGG-AAC-GCC-GGC-GCT              3'
```

qui code pour le peptide signal-peptide de formule (1) :
M A P S G K S T L L L L F L L L C L P S W N A G A
et

```
5'    ATG-AAA-TCC-ACG-CTG-CTT-CTC-TTA-TTT-CTG

      CTC-CTG-TGC-CTG-CCC-TCT-TGG-AAC-GCC-GGC

      GCT                          3'
```

qui code pour le peptide-signal de formule (2) :
M K S T L L L L L F L L L C L P S W N A G A

L'invention concerne encore les vecteurs d'expression portant une séquence d'ADN codant pour un précurseur d'un polypeptide caractérisé en ce que la portion de cette séquence codant pour le peptide-signal est une séquence selon l'invention.

Le peptide-signal selon l'invention, les séquences d'ADN codant pour lui et les vecteurs d'expression portant ces séquences trouvent leur application dans la production périplasmique de polypeptides par les bactéries répondant de manière négative au test de coloration de Gram (bactéries dites gram-négatives) transformées par ces vecteurs.

L'invention concerne donc également les bactéries gram-négatives transformées par les vecteurs précédemment définis. Parmi celles-ci on apprécie celles qui appartiennent à l'espèce <u>Escherichia coli</u>. De préférence ces dernières portent une ou plusieurs mutations si possible stables, par exemple des mutations par délétion, affectant le gène cya et/ou le gène crp.

L'invention concerne selon un autre aspect un procédé de production périplasmique d'un polypeptide consistant à cultiver des cellules de bactéries gram-négatives précédemment définies, à soumettre les cellules à un choc osmotique et à séparer le polypeptide recombinant du surnageant du choc osmotique.

Le procédé selon l'invention permet une production aussi bien selon un mode inductible, lorsque l'expression de la séquence d'ADN codant pour le précurseur est placée sous le contrôle d'un promoteur inductible que selon un mode constitutif, où la production du polypeptide est continue dés la mise en culture de la souche transformée.

Le procédé selon l'invention convient à la production de toutes sortes de polypeptides hétérologues par rapport à la souche mise en oeuvre. Il est ainsi adapté à la production de polypeptides d'origine eucaryote. Il peut s'agir de protéines à proprement parler, telles que notamment l'hormone de croissance humaine (hGH) ou de peptides de plus petite taille, tels que notamment une forme naturelle ou un variant de l'hirudine, par exemple le variant HV2 ($Lys^{47}$).

La présente invention va être maintenant décrite plus en détail à l'aide des trois exemples ci-après, dans lesquels il sera fait référence aux cinq figures annexées.

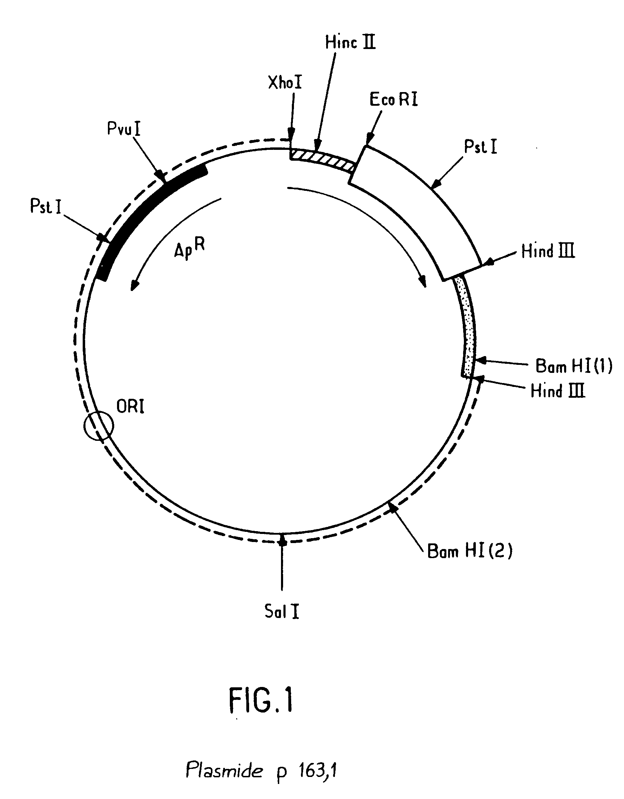

La figure 1 représente une carte de restriction du plasmide p163,1. Les différents segments de restriction sont marqués de manière arbitraire selon la légende ci-dessous :

```
  ———————⊖———— = Localisation de l'origine de réplication (ORI)
  ——————     ————
```

_____
-- -- -- -- -- -- --  = Segment d'ADN issu du plasmide pBR322


_____
_____  = Segment d'ADN contenant la séquence codant pour
un précurseur naturel de l'hGH


▒▒▒▒▒▒▒▒▒▒  = Segment d'ADN du phage fd contenant un terminateur de transcription


////////////  = Segment d'ADN contenant un promoteur-opérateur
hybride tryptophane-lactose UV5


████████  = Segment d'ADN codant pour la β-lactamase (ApR :
résistance à l'ampicilline)


La figure 2 représente la carte de restriction du plasmide p160,1 dont les fragments PvuI-XhoI-BamHI(1) et PvuI-ORI-bamHI(2) proviennent respectivement des plamides p163,1 et pBR327 et dont le petit fragment BamHI(2)-BamHI(1) est le fragment 3 décrit dans l'exemple 1 ci-après.

La figure 3 représente une carte de restriction du plasmide p373,2. Les différents segments de restriction sont marqués de manière arbitraire selon la légende ci-dessous :


_____
·+ — + — + — +  = Séquence PvuI-BamHI (2) issue du plasmide pBR327


_____
-- -- -- -- -- -- --  = Séquence PvuI-XhoI issue du plasmide p163,1

////////// = Séquence XhoI-HincII issue du plasmide p163,1

ClaI
NdeI PstI
(HincII) |
●●●●●● = Fragment 4 décrit dans l'exemple 1 ci-après

XXXXXXXXX = Fragment 3 décrit dans l'exemple 1 ci-après

:::::::::::::: = Segment d'ADN du phage fd contenant un terminateur de transcription

_____
_____ = Fragment PstI-HindIII du segment d'ADN contenant la séquence codant pour le précurseur
naturel de l'hGH.

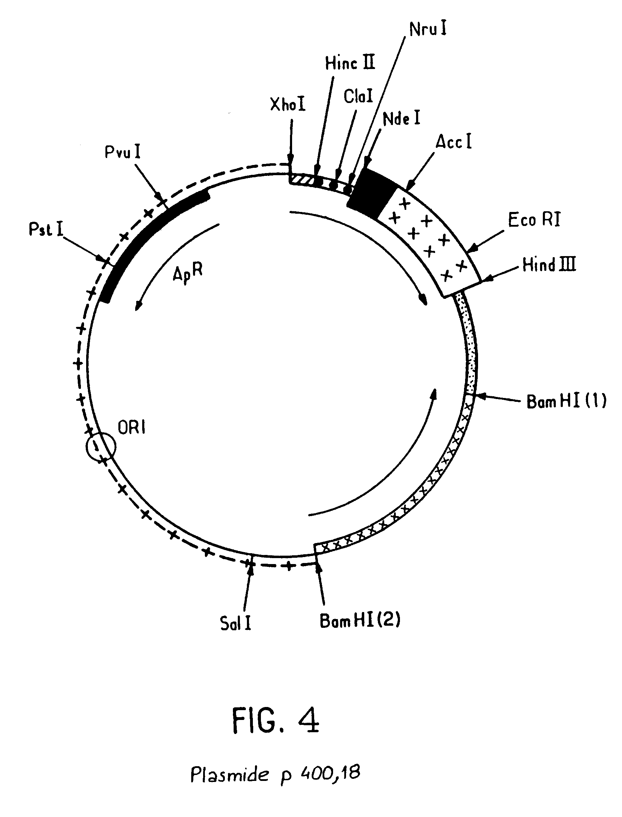

La figure 4 représente la carte de restriction du plasmide p400,18. Les différents fragments de restriction sont définis de manière arbitraire selon la légende ci-dessous :

_____
+ ─ + ─ + ─ + = Séquence PvuI-BamHI (2) issue du plasmide pBR327

_____
─ ─ ─ ─ ─ ─ ─ = Séquence PvuI-XhoI issue du plasmide p163,1

////////// = Séquence XhoI-HincII issue du plasmide p163,1

XXXXXXXX = Fragment 3 décrit dans l'exemple 1 ci-après

:::::::::::::: = Segment d'ADN du phage fd contenant un terminateur de transcription

■●■●■●■● = segment d'ADN comprenant en particulier le promoteur du précurseur de l'hirudine (partie
HincII-NdeI du fragment 4)

⬛ X X X X / X X X = Séquence codante du précurseur de l'hirudine
comprenant la séquence codant pour le peptide-
signal de formule (1) représentée par la partie
noire de ce symbole (réunion des fragments 5 et
7)

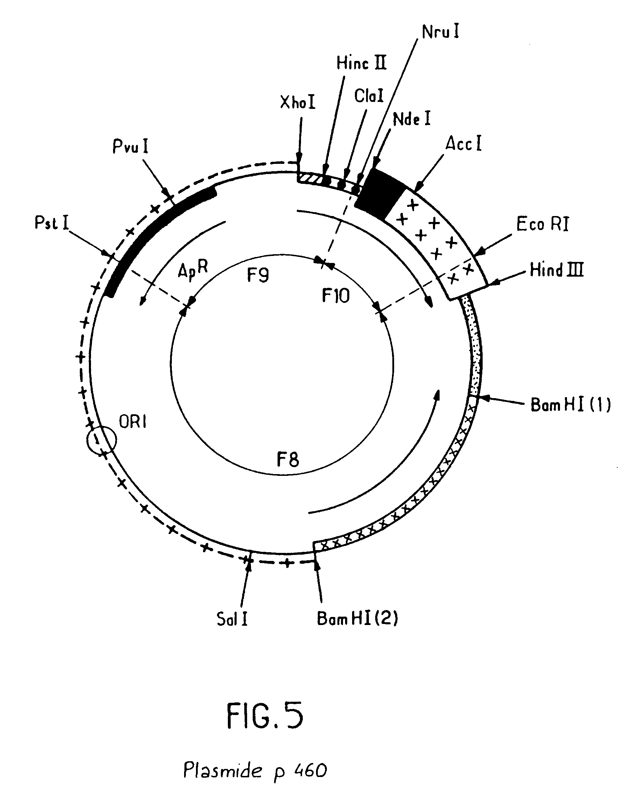

La figure 5 représente la carte de restriction du plasmide p460. Les différents fragments son définis de manière arbitraire selon la légende ci-dessous :

⎺⎺⎺⎺⎺⎺⎺
+ — + — + — + = Séquence PvuI-BamHI (2) issue du plasmide pBR327

⎯⎯⎯⎯⎯⎯⎯
— ⎼ — ⎯ — ⎯ — = Séquence PvuI-XhoI issue du plasmide p163,1

//////////// = Séquence XhoI-HincII issue du plasmide p163,1

XXXXXXXX = Fragment 3 décrit dans l'exemple 1 ci-après

⠿⠿⠿⠿⠿⠿ = Segment d'ADN du phage fd contenant un terminateur de transcription

●●●●●● = Segment d'ADN comprenant en particulier le promoteur du précurseur de l'hirudine (partie
HincII-NdeI du fragment 4)

```
    ┌─────┬─────────┐
    │█████│ X X X X │   = Séquence codante du précurseur de l'hirudine
    │█████│ X X X X │
    └─────┴─────────┘     comprenant  la séquence codant pour le  peptide-
```

signal de formule (2) représentée par la  partie

noire de ce symbole.

EXEMPLE 1 : **Production périplasmique d'hormone de croissance humaine avec le peptide-signal de formule (1) :**

M A P S G K S T L L L L F L L L C L P S W N A G A

Une souche de l'espèce <u>Escherichia coli</u> directement apparentée à la souche, décrite dans la demande de brevet EP-A-0245138 et déposée à la Collection Nationale de Cultures de Microorganismes (CNCM, Paris, France) le 17 Février 1986 sous la référence I-529, est mise en oeuvre. Cette souche porte une mutation cya par délétion et une mutation crp par délétion.

Un plasmide portant une séquence d'ADN codant pour un précurseur de l'hGH dont le peptide-signal est celui de l'invention de formule (1) : M A P S G K S T L L L L F L L L C L P S W N A G A a été préparé. Ce plasmide a été dénommé p398.

## 1. Construction du plasmide p398

### 1a) Construction du plasmide p373,2

La stratégie mise en oeuvre a fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS EF, FRITSCH et J. SAMBROOK dans "Molecular cloning, a laboratory manual" (Cold Spring Harbor Laboratory, 1984). La synthèse des oligonucléotides est réalisée à l'aide d'un synthétiseur d'ADN Biosearch 4 600.

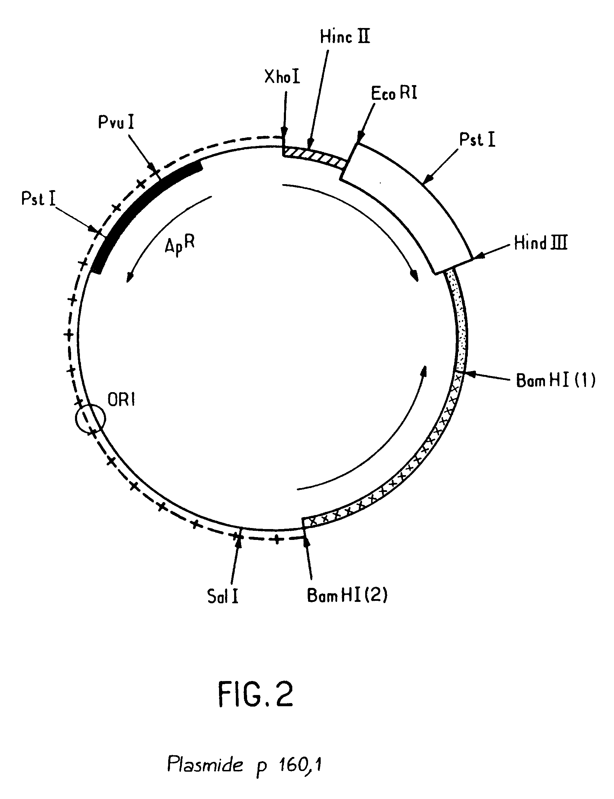

On a soumis le plasmide p163,1 (figure 1), décrit dans la demande de brevet EP-A-0245138 (représenté sur la figure 2 de ce document, laquelle ne fait pas apparaître le site BamHI (1) représenté sur la figure 1 de la présente demande) et présent dans la souche déposée à la CNCM sous la référence I-530 le 17 Février 1986, à une digestion par les enzymes PvuI et BamHI. Ce plasmide contient le gène codant pour l'hGH. Le fragment PvuI-BamHI (1) - ci-après fragment 1 - contenant le site d'action de l'enzyme de restriction XhoI, représenté sur la figure 1, a été purifié.

De même, on a soumis le plasmide pBR327, bien connu de l'homme de l'art, (cf. SOBERON, X et al., Gene, 9 (1980) 287-305) à une digestion par les enzymes PvuI et BamHI. Le fragment PvuI-BamHI (2) - ci-après fragment 2 -, contenant l'origine de réplication, a été purifié.

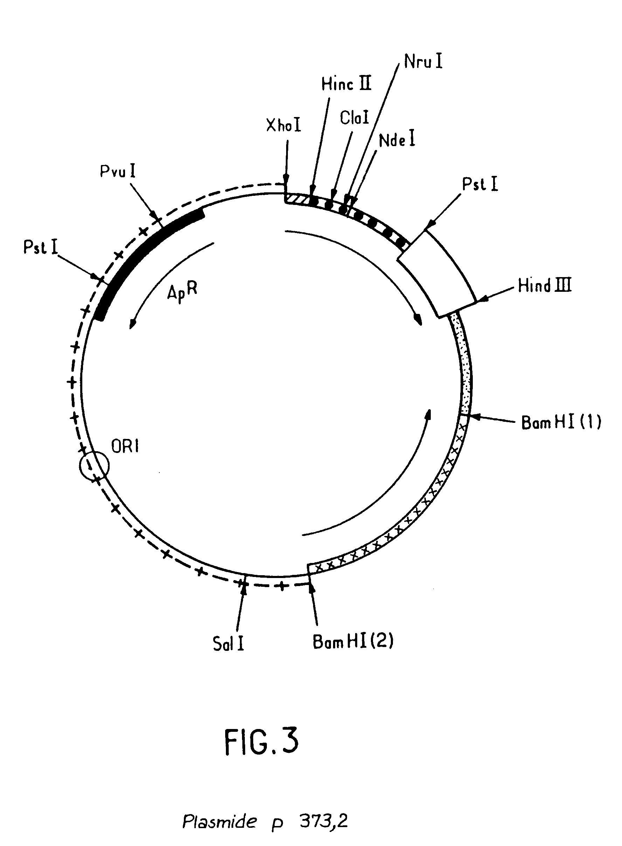

Puis on a préparé le fragment 3, qui est un fragment synthétique BamHI(1)-BamHI(2) contenant le gène lac i et son promoteur dont la séquence est la suivante sur laquelle les deux extrémités du brin sont repérées par les nombres 1 et 2 pour préciser l'orientation du fragment dans les plasmides décrits aux figures 2 et 3.

## FRAGMENT 3

BamHI(1)

```
5'          GATCC   GCGGAAGCAT  AAAGTGTAAA  GCCTGGGGTG  CCTAATGAGT
         GAGCTAACTT  ACATTAATTG  CGTTGCGCTC  ACTGCCCGCT  TTCCAGTCGG
         GAAACCTGTC  GTGCCAGCTG  CATTAATGAA  TCGGCCAACG  CGCGGGGAGA
         GGCGGTTTGC  GTATTGGGCG  CCAGGGTGGT  TTTTCTTTTC  ACCAGTGAGA
         CGGGCAACAG  CTGATTGCCC  TTCACCGCCT  GGCCCTGAGA  GAGTTGCAGC
         AAGCGGTCCA  CGCTGGTTTG  CCCCACCACC  CGAAAATCCT  GTTTGATGGT
         GGTTAACGGC  GGGATATAAC  ATGAGCTGTC  TTCGGTATCG  TCGTATCCCA
         CTACCGAGAT  ATCCGCACCA  ACGCGCAGCC  CGGACTCGGT  AATGGCGCGC
         ATTGCGCCCA  GCGCCATCTG  ATCGTTGGCA  ACCAGCATCG  CAGTGGGAAC
         GATGCCCTCA  TTCAGCATTT  GCATGGTTTG  TTGAAAACCG  GACATGGCAC
         TCCAGTCGCC  TTCCCGTTCC  GCTATCGGCT  GAATTTGATT  GCGAGTGAGA
         TATTTATGCC  AGCCAGCCAG  ACGCAGACGC  GCCGAGACAG  AACTTAATGG
         GCCCGCTAAC  AGCGCGATTT  GCTGGTGACC  CAATGCGACC  AGATGCTCCA
         CGCCCAGTCG  CGTACCGTCT  TCATGGGAGA  AAATAATACT  GTTGATGGGT
         GTCTGGTCAG  AGACATCAAG  AAATAACGCC  GGAACATTAG  TGCAGGCAGC
         TTCCACAGCA  ATGGCATCCT  GGTCATCCAG  CGGATAGTTA  ATGATCAGCC
         CACTGACGCG  TTGCGCGAGA  AGATTGTGCA  CCGCCGCTTT  ACAGGCTTCG
         ACGCCGCTTC  GTTCTACCAT  CGACACCACC  ACGCTGGCAC  CCAGTTGATC
         GGCGCGAGAT  TTAATCGCCG  CGACAATTTG  CGACGGCGCG  TGCAGGGCCA
         GACTGGAGGT  GGCAACGCCA  ATCAGCAACG  ACTGTTTGCC  CGCCAGTTGT
         TGTGCCACGC  GGTTGGGAAT  GTAATTCAGC  TCCGCCATCG  CCGCTTCCAC
         TTTTTCCCGC  GTTTTCGCAG  AAACGTGGCT  GGCCTGGTTC  ACCACGCGGG
         AAACGGTCTG  ATAACAGACA  CCGGCATACT  CTGCGACATC  GTATAACGTT
         ACTGGTTTCA  CATTCACCAC  CCTGAATTGA  CTCTCTTCCG  GGCGCTATCA
         TGCCATACCG  CGAAAGGTTT  TGCGCCATTC  GATGGTGTCC  G          3'
                                                            BamHI(2)
```

Les fragments 1, 2 et 3 ont été alors ligués de manière à obtenir le plasmide p160,1 représenté sur la figure 2.

Ce plasmide a été soumis à une digestion partielle par les enzymes de restriction HincII et PstI. Le grand fragment HincII-PstI, contenant l'origine de réplication et représenté sur la figure 2, a été ensuite ligué au fragment 4, représenté ci-après, qui est un fragment synthétique d'ADN portant une séquence codant pour les 44 premiers acides aminés d'un précurseur naturel de l'hGH et en amont de cette séquence des signaux de régulation.

## FRAGMENT 4

```
                                                              ClaI

        5'       TCGAGCTGACTGACCTGTTGCTTATATTACATCGA
                 ------------------------------------
                 AGCTCGACTGACTGGACAACGAATATAATGTAGCT

                                                              NdeI

TAGCGTATAATGTGTGGAATTGTGAGCGATAACAATTTCACACAGTTTAACTTTAAGAAGGAGATATACAT
----------------------------------------------------------------------
ATCGATATTACACACCTTAACACTCGCCTATTGTTAAAGTGTGTCAAATTGAAATTCTTCCTCTATATGTA

ATG GCT ACC GGA TCC CGG ACT AGT CTG CTC CTG GCT TTT GGC CTG CTC TGC CTG
---------------------------------------------------------------------
TAC CGA TGG CCT AGG GCC TGA TCA GAC GAG GAC CGA AAA CCG GAC GAC ACG GAC
 M   A   T   G   S   R   T   S   L   L   L   A   F   G   L   L   C   L
-26

                                                 XbaI
CCC TGG CTT CAA GAG GGC AGT GCC TTC CCA ACC ATT CCC TTA TCT AGA CTT TTT
---------------------------------------------------------------------
GGG ACC GAA GTT CTC CCG TCA CGG AAG GGT TGG TAA GGG AAT AGA TCT GAA AAA
 P   W   L   Q   E   G   S   A   F   P   T   I   P   L   S   R   L   F
                         -1   1

GAC AAC GCT ATG CTC CGC GCC CAT CGT CTG CAC CAG CTG GCC TTT GAC ACC TAC
---------------------------------------------------------------------
CTG TTG CGA TAC GAG GCG CGG GTA GCA GAC GTG GTC GAC CGG AAA CTG TGG ATC
 D   N   A   M   L   R   A   H   R   L   H   Q   L   A   F   L   T   Y

                                                              PstI
CAG GAG TTT GAA GAA GCC TAT ATC CCA AAG GAA CAG AAG TAT TCA TTC CTG CA
-------------------------------------------------------------------        5'
GTC CTC AAA CTT CTT CGG ATA TAG GGT TTC CTT GTC TTC ATA AGT AAG G
 Q   E   F   E   E   A   Y   I   P   K   E   Q   K   Y   S   F
                                                            44
```

Dans ce fragment, les acides aminés sont désignés par des lettres selon le code suivant :

| | |
|---|---|
| A = Alanine | M = Méthionine |
| C = Cystéine | N = Asparagine |
| D = Acide aspartique | P = Proline |
| E = Acide glutamique | Q = Glutamine |
| F = Phénylalanine | R = Arginine |
| G = Glycine | S = Sérine |
| H = Histidine | T = Thréonine |
| I = Isoleucine | V = Valine |
| K = Lysine | W = Tryptophane |
| L = Leucine | Y = Tyrosine |

Sont successivement soulignées dans ce fragment les séquences -35 (TTGCTT) et -10 (TATAAT) de la séquence promotrice, et la séquence de shine et Dalgarno bien connue de l'homme de l'art.

Le plasmide p380,1 a été ainsi obtenu.

Le plasmide p380,1 a été ensuite soumis à une digestion par les enzymes de restriction ClaI et NdeI de manière à en éliminer le petit fragment ClaI - NdeI du fragment 4 ci-dessus et à lui substituer le fragment ClaI - NdeI ci-après :

```
                    ClaI
      5'        CGATAGCGTATAATGTGTGGAATTGTGAGCGGATAACA
                TATCGCATATTACACACCTTAACACTCGCCTATTGT


                               NruI                    NdeI
                                 ▼
                ATTTCACACAGTTTTTCGCGAAGAAGGAGATATACA
                TAAAGTGTGTCAAAAAGCGCTTCTTCCTCTATATGTAT        5'
                                 ▲
```

Le plasmide résultant est le plasmide p373,2 (figure 3).

**1b) Construction du plasmide p398**

Enfin, le plasmide p373,2 a été soumis à une digestion par les enzymes de restriction NdeI et XbaI de manière à en éliminer le fragment NdeI - XbaI du fragment 4 ci-dessus et à lui substituer le fragment NdeI - XbaI synthétique représenté ci-après.

```
               NdeI
                ┌→
      5'       TATG GCT CCA TCT GGC AAA TCC ACG CTG
                  AC CGA GGT AGA CCG TTT AGG TGC GAC

               CTT CTC TTA TTT CTG CTC CTG TGC CTG
               GAA GAG AAT AAA GAC GAG GAC ACG GAC


               CCC TCT TGG AAC GCC GGC GCT┐TTC CCA
               GGG AGA ACC TTG CGG CCG CGA↓AAG GGT

                                       XbaI
               ACC ATT CCC TTA T
               TGG TAA GGG AAT AGATC     5'
```

Le plasmide p398 ainsi obtenu comporte la séquence d'ADN particulièrement appréciée codant pour le peptide-signal de formule (1). Cette séquence est délimitée ci-dessus par deux flèches.

**2. Méthodologie générale**

Les essais réalisés ont porté sur 6 clones du plasmide p398 (Clones 2, 3, 5, 6, 7 et 8), les résultats étant appréciés par rapport au plasmide p373,2 qui contient une séquence d'ADN codant pour le précurseur naturel de l'hGH. Ils ont consisté à cultiver les couples hôte-vecteur concernés, préalablement préparés (cf. § 2.1), dans des conditions telles qu'une biomasse suffisante soit obtenue (cf. § 2.2), et que les cellules soumises à une induction produisent l'hGH (cf. § 2.3), à recueillir par choc osmotique les protéines contenues dans l'espace périplasmique (cf. § 2.4), à soumettre les bactéries à une lyse totale de manière à disposer d'un extrait protéique total (cf. § 2.5), à doser l'hGH périplasmique recueillie en 2.4 (cf. § 2.6) et à analyser par la technique dite du Western Blot les surnageants obtenus en 2.4 et en 2.5 (cf. § 2.7).

## 2.1 Préparation des couples hôte-vecteur

Les couples hôte-vecteur ont été préparés selon les techniques de transformation bactérienne connues de l'homme de l'art et qui sont décrites notamment dans les ouvrages ci-après.
- Molecular cloning - A Laboratory Manual - T. Maniatis E.F., Fritsch and J. Sambrook - Cold Spring Harbor Laboratory - 1982.
- Experiments in Molecular Genetics - J.H. MILLER - Cold Spring Harbor Laboratory - 1972.

## 2.2 Culture

a) Ensemencement

Une colonie isolée obtenue sur milieu solide, (milieu LB + agar-agar) a été mise en suspension dans 5 ml d'un milieu (milieu LB).
Le milieu LB utilisé a les caractéristiques ci-après :
- ses composants introduits avant autoclavage sont :

| . de la Bactotryptone | 10 g |
| . de l'extrait de levure | 5 g |
| . du chlorure de sodium | 5 g |
| . de l'eau distillée qsp | 1 l |

- son pH est ajusté à 7,3 avant autoclavage
- de l'ampicilline est ajoutée après autoclavage à raison de 100 $\mu$g/ml

b) Incubation

La suspension préparée en a) a été placée à 37°C pendant 18 h de façon que la culture arrive en phase de croissance stationnaire. La suspension dense obtenue a été diluée en milieu LB de manière à obtenir une valeur de densité optique lue à 600 nm - DO à 600 nm - proche de 0,03 puis 25 ml de cette suspension bactérienne ont été mis en incubation à 37°C sous agitation jusqu'à obtention d'une DO à 600 nm voisine de 0,3.

## 2.3 Induction

A la suspension bactérienne obtenue selon 2.2.b, on a ajouté de l'isopropyl-$\beta$-D-thiogalactose (ou IPTG) en quantité telle que sa concentration finale soit égale à 1 mM ; l'IPTG a été ici utilisé pour provoquer le déclenchement et le maintien de la synthèse du précurseur de l'hGH en neutralisant l'action du répresseur venant normalement se fixer sur l'opérateur-Lactose.
La suspension additionnée d'IPTG a été maintenue sous agitation à 37°C pendant 2 h 30.

## 2.4 Choc osmotique

On s'est référé au protocole décrit par N.G. NOSSAL et L.A. HEPPEL dans "The Journal of Biological Chemistry 241 (1966) 3055-3063".

a) Lavage au Tris et à l'EDTA

Un échantillon de la suspension, telle qu'obtenue en 2.3. après induction, a été prélevé et centrifugé 5 minutes à 6 000 g.
Le culot a été repris dans un volume de tampon à pH 7 (solution A) (cf. ci-dessus) de façon que la suspension obtenue présente une DO à 600 nm voisine de 10.
Le tampon utilisé a été préparé par addition dans de l'eau distillée de :
. tri(hydroxyméthyl)aminométhane-HCl ou tris-HCl ajouté de façon à avoir une concentration finale de 30 mM.
. acide éthylènediaminetétraacétique ou EDTA ajouté de façon à avoir une concentration finale de 1 mM.

b) Action du saccharose

La suspension obtenue en 2.4.a. a été centrifugée 5 minutes à 6 000 g.

Le culot a été repris délicatement à volume constant dans une solution B préparée extemporanément et correspondant à la solution A à laquelle est ajouté du saccharose à raison de 15 g pour 100 ml.

La suspension a été laissée 10 minutes à 20°C. Puis elle a été centrifugée 5 minutes à 6 000 g. Les tubes de centrifugation ont été placés dans de la glace fondante.

On a éliminé avec soin le surnageant et on lui a substitué (à volume constant) de l'eau désionisée et portée préalablement à la température de la glace fondante.

La suspension ainsi préparée (dont la DO à 600 nm était voisine de 10) a été laissée 5 minutes à 0°C.

c) Recueil des protéines à localisation périplasmique

La suspension obtenue en 2.4.b. a été centrifugée 10 minutes à 18 000 g.

Le surnageant qui contenait les protéines à localisation périplasmique a été recueilli.

## 2.5 Lyse totale

Un échantillon de la suspension, telle qu'obtenue en 2.3. après induction, a été prélevé et centrifugé dans un tube Eppendorf 5 minutes à 6 000 g.

Le culot a été remis en suspension dans un volume de tampon tel que 1 ml de suspension présente une DO à 600 nm de 0,2.

Le tampon a été préparé à partir d'un tampon concentré 2 fois comprenant une solution dans l'eau distillée :
- du Tris-HCl 0,125 M, pH 6,8 ;
- du dodécylsulfate de sodium (4 % (p/v)) ;
- du glycérol (20 % (p/v)) ;
- du $\beta$-mercaptoéthanol (10 % (p/v)) ;
- du bleu de bromophénol (0,02 % (v/v)).

Le tube a été placé 10 minutes au bain-marie réglé à 100°C puis la suspension a été centrifugée pendant 5 minutes à 6 000 g et le surnageant a été recueilli.

## 2.6 Dosage de l'hGH périplasmique

Le surnageant obtenu en 2.4.c. a été soumis à une chromatographie de type liquide haute pression à l'aide d'un appareillage muni d'un système d'injection calibré et équipé d'un détecteur réglé sur 220 nm.

On a utilisé :
. une colonne phase inverse C 8 -300 Angströms, en acier, de 10 cm de longueur et de 4,6 mm de diamètre interne (SYNCHROM référence C 8 R103-10),
. une phase mobile constituée par un gradient linéaire de 20 minutes passant respectivement de 70 volumes de solution S1 et 30 volumes de solution S2 à 40 volumes de solution S1 et 60 volumes de solution S2.

Les solutions S1 et S2 avaient les caractéristiques suivantes :
. S1 = eau purifiée contenant 0,1 % (v/v) d'acide trifluoroacétique,
. S2 = acétonitrile pour HPLC contenant 0,08 % (v/v) d'acide trifluoroacétique.

Le débit était de 1 ml par minute.

La densité optique des fractions a été mesurée et la quantité d'hGH périplasmique exprimée en microgrammes par ml de surnageant a été déterminée par comparaison avec une gamme étalon préétablie.

## 2.7. Analyse par la technique dite du Western Blot

Il a été procédé successivement aux opérations suivantes :
- séparation électrophorétique sur gel (selon le protocole décrit par LAEMMLI, U.K., Nature 227 (1970) 680-685), des différentes protéines contenues dans chacun des surnageants obtenus selon 2.4.c. et 2.5 ; le gel utilisé était un gel de polyacrylamide (15 % p/v) contenant 0,5 % de dodécylsulfate de sodium ;
- transfert desdites protéines contenues dans le gel sur un filtre de nitrocellulose (selon la technique de H. TOWBIN et al. Proc. Natl. Acad. Scie. USA 76 (1979) 4350-4354) ;

- immunodétection réalisée selon la technique de BURNETTE (W.W. BURNETTE Anal. Biochem. 112 - (1981) 195-203) ; elle implique successivement :

  . le rinçage du filtre de nitrocellulose 10 minutes avec un tampon A (Tris-HCl 10 mM, NaCl 170 mM, KI 1 mM) ;

  . la mise en contact du filtre de nitrocellulose pendant 30 minutes à 37ºC avec un tampon B (tampon A additionné de sérum-albumine bovine à raison de 3 g pour 100 ml) ;

  . la mise en contact du filtre de nitrocellulose pendant 18 h à 20ºC avec un immun-sérum (un anticorps polyclonal reconnaissant l'hGH mature et son précurseur) ;

  . le rinçage du filtre de nitrocellulose avec le tampon B ;

  . la mise en contact du filtre de nitrocellulose pendant 6 h à 20ºC avec une solution de protéine A marquée à l'iode 125 à 0,1 microcurie par ml ;

  . le rinçage du filtre avec le tampon A ;

  . le séchage du filtre entre deux feuilles absorbantes ;

  . la mise au contact d'un film radiographique ;

  . la révélation du film.

## 3. RESULTATS

### 3.1 Dosage de l'hGH périplasmique

Les résultats sont reportés dans le tableau ci-après :

|  | PLASMIDE TESTE | | | | | | |
|---|---|---|---|---|---|---|---|
|  | Témoin | PLASMIDE 398 | | | | | |
|  | 373,2 | 398,2 | 398,3 | 398,5 | 398,6 | 398,7 | 398,8 |
| hGH périplasmique exprimé en microgrammes par ml de surnageant recueilli après choc osmotique et ramené à une turbidité telle que DO à 600 nm = 1 | 1,5 | 2,5 | 2,9 | 2,8 | 3,1 | 3,1 | 3,4 |

Il apparaît clairement que le plasmide 398 permet une production périplasmique multipliée environ par deux par rapport à celle que permet le plasmide 373,2.

### 3.2 Analyse par la technique dite du Western Blot

L'analyse des films autoradiographiques révèle que le précurseur n'a pas été détecté dans les extraits obtenus après lyse totale des bactéries transformées avec le plasmide p398 alors qu'il est détecté dans les extraits obtenus après lyse totale des bactéries du clone p373,2. Ceci montre que le peptide-signal selon l'invention est à même de permettre, avec une grande efficacité, le franchissement par le précurseur de la membrane cytoplasmique et sa maturation concomitante.

Ces résultats soulignent le grand intérêt de l'utilisation du peptide-signal de formule (1) selon l'invention pour la production périplasmique d'une protéine telle que l'hormone de croissance humaine.

## EXEMPLE 2 Production périplasmique d'un variant de l'hirudine avec le peptide-signal de formule (1) :

M A P S G K S T L L L L F L L L C L P S W M A G A

### 1. Souche et plasmide

La souche décrite à l'exemple 1 a été utilisée.

Un plasmide dénommé p400 a été construit à partir du plasmide p373,2. Il porte une séquence d'ADN codant pour le variant $(Lys^{47})HV2$ décrit dans EP-A-0273800 et dont la formule est ci-après rappelée :

```
ILE THR TYR THR ASP CYS THR GLU SER GLY GLN ASN LEU CYS LEU

CYS GLU GLY SER ASN VAL CYS GLY LYS GLY ASN LYS CYS ILE LEU

GLY SER ASN GLY LYS GLY ASN GLN CYS VAL THR GLY GLU GLY THR

PRO LYS PRO GLU SER HIS ASN ASN GLY ASP PHE GLU GLU ILE PRO

GLU GLU TYR LEU GLN
```

précédée d'une séquence codant pour le peptide-signal selon l'invention de formule (1).

**Construction du plasmide p 400**

Un fragment synthétique AccI-HindIII (fragment 5) contenant une séquence codant pour ce variant (sauf les 3 premiers acides aminés) a été préparé. Il est représenté ci-dessous, une flèche désignant le codon correspondant au dernier acide aminé :

## FRAGMENT 5

```
        AccI
5'      AT ACA GAC TGC ACA GAA TCG GGT
           TGT CTG ACG TGT CTT AGC CCA


        CAA ATT TTG TGC CTC TGC GAG GGA AGC AAT
        GTT TTA AAC ACG GAG ACG CTC CCT TCG TTA


        GTT TGC GGT AAA GGC AAT AAG TGC ATA TTG
        CAA ACG CCA TTT CCG TTA TTC ACG TAT AAC


        GGT TCT AAT GGA AAG GGC AAC CAA TGT GTC
        CCA AGA TTA CCT TTC CCG TTG GTT ACA CAG
```

15

```
ACT GGC GAA GGT ACA CCG AAA CCT GAA AGC
TGA CCG CTT CCA TGT GGC TTT GGA CTT TCG


CAT AAT AAC GGC GAT TTC GAA GAA ATT CCA
GTA TTA TTG CCG CTA AAG CTT CTT TAA GGT


GAA GAA TAT TTA CAA TGA AAA ATG AAA GAA
CTT CTT ATA AAT GTT ACT TTT TAC TTT CTT
                                    Eco RI
                                      ▼
TAT CAA TCA TAG AGA ATT TTG ATT TGG GAA
ATA GTT AGT ATC TCT TAA AAC TAA ACC CTT


TTC GAA AAG TTC GCT GGT ACT AAG GCT TTG
AAG CTT TTC AAG CGA CCA TGA TTC CGA AAC
 ▲

TTA GAC GAA GTT GTC AAG AGC TCT GCT GCT
AAT CTG CTT CAA CAG TTC TCG AGA CGA CGA


GGT AAC ACC GTC ATC ATT GGT GGT GGT GAC
CCA TTG TGG CAG TAG TAA CCA CCA CCA CTG


ACT GCC ACT GTC GCT AAG AAG TAC GGT GTC
TGA CGG TGA CAG CGA TTC TTC ATG CCA CAG
                        HindIII
ACT GAC AAG ATC CCA
TGA CTG TTC TAG GGT TCG A -    5'
```

Le plasmide p373,2 a été soumis à une digestion par les enzymes de restriction NdeI et HindIII et le fragment NdeI-HindIII (fragment 6) contenant l'origine de réplication, tel que représenté à la figure 3, a été purifié.

Un fragment synthétique NdeI-AccI (fragment 7) dont la séquence est donnée ci-dessous a été préparé.

FRAGMENT 7

```
NdeI
5' TATGGCTCCATCTGGCAAATCCACGCTGCTTCTCTTATTTCTGCTCCTGTGCCTGCCCTCT-
    ACCGAGGTAGACCGTTTAGGTGCGACGAAGAGAATAAAGACGAGGACACGGACGGGAGA-


                        AccI
    TGGAACGCCGGCGCTATTACGT
    ACCTTGCGGCCGCGATAATGCATA   5'
```

Ce fragment contient une séquence d'ADN préférée codant pour le peptide-signal de formule (1) délimitée par deux flèches et suivie de nucléotides correspondant aux 3 premiers codons du variant de l'hirudine (Lys$^{47}$)HV2.

Les fragments 5, 6 et 7 ont été ligués ; le plasmide obtenu est le plasmide p400, représenté sur la figure 4.

## 2. Méthodologie générale

Le plasmide p400 a été introduit par transformation dans la souche bactérienne.

Les essais ont été réalisés en parallèle sur deux clones distincts (clones p400,18 et p400,24) et conformément au mode opératoire décrit aux paragraphes 2.1 et 2.2 de l'exemple 1. Les cultures ont été induites selon la méthode indiquée au paragraphe 2.3 de l'exemple 1 mais en y apportant deux aménagements : l'induction a été initiée par l'addition d'IPTG lorsque la culture a atteint une DO à 600 nm d'environ 0,5 et elle a été maintenue pendant 3h30 pour un premier essai et 17 h pour un second essai.

Après induction, les cellules ont été soumises à un choc osmotique (cf. § 2.4, exemple 1) et l'activité antithrombine de l'hirudine du surnageant recueilli a été mesurée.

La détermination de cette activité a fait appel à la technique décrite par Markwardt, F. et al. (Thromb. Haemostas. 52 (19) 160-163) et précisée par Harvey, R.P. et al. (Proc. Natl. Acad. Sci. USA 83 (1986) 1084-1088).

Le variant de l'hirudine obtenu à partir de l'un des clones a été purifié par chromatographie liquide haute pression et sa séquence NH$_2$-terminale a été déterminée.

## 3. RESULTATS

Les résultats sont reportés sur le tableau ci-après.

Ils sont exprimés en unités antithrombine par ml de surnageant recueilli après choc osmotique et ramené à une turbidité telle que DO à 600 nm = 10.

|  |  | Plasmide p 400 | |
| --- | --- | --- | --- |
|  |  | clone p400,18 | clone p400,24 |
| Durée de l'induction | 3h.30 | 241 | 369 |
|  | 17h | 1595 | 983 |

Les valeurs d'activité antithrombine spécifique de l'hirudine connues étant comprises entre 13000 et 17700 unités antithrombine par mg d'hirudine (Loison, G. et al., Bio/Technology 6:72-77 (1988)), on peut en déduire que l'on a extrait après 17 h d'induction de 5 à 10 mg d'hirudine par litre de surnageant à DO à 600 nm = 1.

Une telle quantité est bien plus élevée que celle que Dodt J. et al. FEBS, 202 (1986) 373-377 ont décrite avec le variant HV1 de l'hirudine produit de façon périplasmique dans une souche d'E. coli transformée à l'aide d'un plasmide portant une séquence codant pour un précurseur hybride de l'hirudine dont le peptide-signal est celui de la phosphatase alcaline.

Il a d'autre part été constaté que l'hirudine produite par le clone p40C,18 a bien l'extrémité NH$_2$-terminale caractéristique du variant (Lys$^{47}$)HV2.

Ces résultats montrent que le peptide-signal selon l'invention de formule 1 est approprié à une production périplasmique efficace d'un peptide tel que le variant (Lys$^{47}$)HV2 de l'hirudine.

**Exemple 3 : Production périplasmique d'un variant de l'hirudine à l'aide du peptide-signal de formule :**

M K S T L L L L F L L L C L P S W N A G A

**1. Souche et plasmide**

La souche décrite à l'exemple 1 a été utilisée.

La stratégie mise en oeuvre pour construire le plasmide p460 qui comprend une séquence codant pour le variant (Lys$^{47}$) HV2 de l'hirudine décrit dans EP-A-0273800, précédée d'une séquence codant pour le peptide-signal selon l'invention de formule (2) :

M K S T L L L L F L L L C L P S W N A G A

fait appel à des fragments d'ADN obtenus à partir du plasmide p400,18 décrit dans l'exemple 2 et à un fragment obtenu après mutagénèse dirigée dans le phage M13mp19 commercialisé par AMERSHAM.

**Construction du plasmide p460**

1a) fragments obtenus à partir du plasmide p400,18.

α) On a soumis le plasmide p400,18 à une digestion par les enzymes Pst et EcoRI. Le fragment Pst-Eco RI de 3868 pb contenant l'origine de réplication ci-après appelé fragment 8 (représenté sur la figure 5 par F8), a été purifié.

β) On a soumis le plasmide p400,18 à une digestion par les enzymes NruI et Pst I. Le petit fragment NruI-PstI de 1062 pb contenant le promoteur, ci-après appelé fragment 9 (représenté sur la figure 5 par F9), a été purifié.

1b) fragment NruI-EcoRI obtenu à partir du phage M13mp19

α) Le fragment XhoI-EcoRI de 650 pb, issu du plasmide p400,18 par digestion à l'aide des enzymes XhoI et EcoRI et purification, qui contient la séquence codant pour le peptide signal de formule (1) : a été inséré dans le polylinker (polysite de clonage) du phage M13mp19 (AMERSHAM) aux sites de restriction SalI/EcoRI. La ligation des sites XhoI et SalI a entraîné la disparition de ces deux sites.

β) Un oligonucléotide de 63 nucléotides de séquence :

```
5'   ATG-AAA-TCC-ACG-CTG-CTT-CTC-TTA-TTT-CTG
     CTC-CTG-TGC-CTG-CCC-TCT-TGG-AAC-GCC-GGC
     GCT            3'
```

a été synthétisé. Cette séquence code pour le peptide-signal de formule (2).

On a utilisé pour construire un fragment muté, en ce qui concerne la séquence codant pour le peptide-signal, par rapport au fragment obtenu en α) la technique de mutagénèse dirigée in vitro, mise en oeuvre à l'aide du kit AMERSHAM 1523. Cette technique, décrite en détail dans le livret accompagnant ce kit consiste en l'introduction d'un fragment XhoI-EcoRI de 650 pb (cf paragraphe α) ci-dessus) dans la forme double brin du phage M13mp19, la purification de la forme simple brin de ce phage recombinant, l'hybridation de l'oligonucléotide de 63 nucléotides mentionné ci-dessus, l'action de DNA polymérase de Klenoω puis de ligase de T4 afin d'obtenir une forme circulaire double brin du phage recombinant dont l'un des brins porte la mutation souhaitée.

γ) Le phage contenant le fragment d'ADN muté a été soumis à une digestion par les enzymes NruI et EcoRI. Le fragment NruI-EcoRI contenant la séquence codant pour le peptide-signal de formule (2) et celle codant pour le variant (Lys$^{47}$) HV2 de l'hirudine, appelé ci-après fragment 10 (représenté sur la figure 5 par F 10) a été purifié.

Les fragments 8, 9 et 10 ont été ligués ; le plasmide obtenu est le plasmide p460, représenté sur la figure 5.

## 2. Méthodologie générale

Le plasmide p460 a été introduit par transformation dans la souche bactérienne décrite à l'exemple 1.

Les essais ont été réalisés en parallèle sur deux clones distincts (clones p460,2 et p460,4) dans lesquels on a vérifié la présence de la séquence de 63 nucléotides mentionnée ci-dessus, et sur le clone témoin p400,18, conformément au mode opératoire décrit aux paragraphes 2.1 et 2.2 de l'exemple 1. Les cultures ont été induites selon la méthode indiquée au paragraphe 2.3 de l'exemple 1 mais en y apportant deux aménagements : l'induction a été initiée par l'addition d'IPTG lorsque la culture a atteint une DO à 600 nm d'environ 0,5 et elle a été maintenue pendant 2 heures.

Après induction, les cellules ont été soumises à un choc osmotique (cf. paragraphe 2.4, exemple 1) et le variant (Lys$^{47}$) HV2 de l'hirudine ainsi libéré dans le surnageant de culture a été dosé par HPLC.

## Dosage du variant (Lys$^{47}$) HV2 de l'hirudine

Le surnageant obtenu à l'issue du choc osmotique a été soumis à une chromatographie liquide haute pression HPLC, à l'aide d'un appareillage muni d'un système d'injection calibré et équipé d'un détecteur réglé sur 220 nm.

On a utilisé :

. Une colonne phase inverse C 8 -300 A°, en acier, de 7,5 cm de longueur et de 4,6 mm de diamètre interne (BECKMAN Ultrapore référence 238 771).

. Une phase mobile constituée par un gradient linéaire de 10 minutes, passant respectivement de 85 volumes de solution S1 et 15 volumes de solution S2 à 50 volumes de solution S1 et 50 volumes de solution S2.

Les solutions S1 et S2 avaient les caractéristiques suivantes :

. S1 = eau purifiée contenant 0,1% (v/v) d'acide trifluoroacétique.

. S2 = acétonitrile pour HPLC contenant 0,08% (v/v) d'acide trifluoroacétique.

Le débit était de 2 ml par minute.

La densité optique des fractions a été mesurée et la quantité de variant (Lys$^{47}$) HV2 périplasmique, exprimée en milligramme par litre de surnageant, a été déterminée par comparaison avec une solution standard de variant (Lys$^{47}$) HV2.

## 3. RESULTATS

Les résultats sont reportés dans les tableaux ci-après. Ils sont exprimés en mg/l de surnageant recueilli à l'issue du choc osmotique et ramené à une densité optique à 600 nm de l.

**1er essai**

| PLASMIDE TESTE | |
|---|---|
| Témoin<br><br>400,18 | 460,2 |
| Variant (Lys$^{47}$) HV2 de l'hirudine en mg/l de surnageant recueilli à l'issue du choc osmotique et ramené à DO à 600 nm = 1 | |
| 1,3 | 3,1 |

**2ème essai**

| PLASMIDE TESTE | | |
|---|---|---|
| Témoin<br><br>400,18 | 460,2 | 460,4 |
| Variant (Lys$^{47}$) HV2 de l'hirudine en mg/l de surnageant recueilli à l'issue du choc osmotique et ramené à DO à 600 nm = 1 | | |
| 1,3 | 5,2 | 4,5 |

Il apparaît à la lecture des tableaux ci-dessus que les plasmides 460,2 et 460,4 permettent une production périplasmique de variant (Lys$^{47}$) HV2 de l'hirudine, nettement plus importante que celle obtenue à l'aide du plasmide 400,18.

Ces résultats montrent que le peptide-signal selon l'invention de formule (2) est particulièrement appropriée à une production périplasmique efficace d'un peptide tel que le variant (Lys$^{47}$) HV2 de l'hirudine.

**Revendications**

**Revendications pour les Etats contractants suivants : AT, BE, CH, LI, DE, FR, GB, GR, IT, LU, NL, SE**

1. Peptide-signal de formule :

    MXKSTLLLLFLLLCLPSWNAGA

    où

| | |
|---|---|
| A = Alanine | M = Méthionine |
| C = Cystéine | N = Asparagine |
| F = Phénylalanine | P = Proline |
| G = glycine | S = Sérine |
| K = Lysine | T = Thréonine |
| L = Leucine | W = Tryptophane |

et X représente une liaison directe entre M et K, un acide aminé choisi dans l'ensemble des 20 acides aminés du code génétique ou un peptide comportant 2, 3 ou 4 acides aminés choisis chacun indépendamment l'un de l'autre dans l'ensemble des 20 acides aminés du code génétique.

**2.** Peptide-signal selon la revendication 1, caractérisé en ce que X représente une liaison directe entre M et K, ou tout ou partie du peptide de séquence APSG.

**3.** Peptide-signal selon la revendication 2, caractérisé en ce que X représente une liaison directe.

**4.** Peptide-signal selon la revendication 2, caractérisé en ce que X représente le peptide de séquence APSG.

**5.** Séquence d'ADN codant pour le peptide-signal selon l'une quelconque des revendications 1 à 4.

**6.** Séquence selon la revendication 5, caractérisée en ce qu'elle à la formule :

```
5'    ATG-GCT-CCA-TCT-GGC-AAA-TCC-ACG-CTG

      CTT-CTC-TTA-TTT-CTG-CTC-CTG-TGC-CTG

      CCC-TCT-TGG-AAC-GCC-GGC-GCT              3'
```

**7.** Séquence selon la revendication 5, caractérisée en ce qu'elle a la formule :

```
5'    ATG-AAA-TCC-ACG-CTG-CTT-CTC-TTA-TTT

      CTG-CTC-CTG-TGC-CTG-CCC-TCT-TGG-AAC

      GCC-GGC-GCT  -3'
```

**8.** Vecteur d'expression portant une séquence d'ADN codant pour un précurseur d'un polypeptide, caractérisé en ce que la portion de cette séquence codant pour le peptide-signal est une séquence selon l'une quelconque des revendications 5 à 7.

**9.** Bactéries gram-négatives caractérisées en ce qu'elles sont transformées par un vecteur selon la revendication 8.

**10.** Bactéries selon la revendication 9, caractérisées en ce qu'elles appartiennent à l'espéce Escherichia coli.

**11.** Bactéries selon la revendication 10, caractérisées en ce qu'elles portent une mutation Cya par délétion et une mutation crp par délétion.

**12.** Bactéries selon l'une des revendications 9 et 10, caractérisées en ce que le polypeptide est une forme naturelle ou un variant de l'hirudine.

**13.** Bactéries selon la revendication 12, caractérisées en ce que le variant de l'hirudine est le HV2 (Lys$^{47}$).

**14.** Bactéries selon l'une des revendications 9 et 10, caractérisées en ce que le polypeptide est l'hormone de croissance humaine.

**15.** Procédé de production périplasmique d'un polypeptide consistant à cultiver des cellules de bactéries gram-négatives transformées par un vecteur d'expression d'un précurseur d'un polypeptide, à soumettre les cellules à un choc osmotique et à séparer le polypeptide recombinant du surnageant du choc osmotique, caractérisé en ce que les bactéries gram-négatives sont des bactéries selon l'une des revendications 9 à 14.

**Revendications pour L'Etat contractant suivant : ES**

1. Procédé de production périplasmique d'un polypeptide caractérisé en ce qu'il consiste à cultiver des bactéries gram-négatives transformées par un vecteur d'expression codant pour un précurseur dudit polypeptide, ledit précurseur comprenant un peptide-signal de formule :

   MXKSTLLLLFLLLCLPSWNAGA

   | | |
   |---|---|
   | A = Alanine | M = Méthionine |
   | C = Cystéine | N = Asparagine |
   | F = Phénylalanine | P = Proline |
   | G = glycine | S = Sérine |
   | K = Lysine | T = Thréonine |
   | L = leucine | W = Tryptophane |

   et X représente une liaison directe entre M et K, un acide aminé choisi dans l'ensemble des 20 acides aminés du code génétique ou un peptide comportant 2, 3 ou 4 acides aminés choisis chacun indépendamment l'un de l'autre dans l'ensemble des 20 acides aminés du code génétique, à soumettre les cellules à un choc osmotique et à séparer le polypeptide recombinant du surnageant du choc osmotique.

2. Procédé selon la revendication 1, caractérisé en ce que X représente une liaison directe entre M et K, ou tout ou partie du peptide de séquence APSG.

3. Procédé selon la revendication 2, caractérisé en ce que X représente une liaison directe.

4. Procédé selon la revendication 2, caractérisé en ce que X représente le peptide de séquence APSG.

5. Procédé selon la revendication 1, caractérisé en ce que la séquence d'ADN codant pour ledit peptide signal a la formule :

   5' ATG-GCT-CCA-TCT-GGC-AAA-TCC-ACG-CTG

   CTT-CTC-TTA-TTT-CTG-CTC-CTG-TGC-CTG

   CCC-TCT-TGG-AAC-GCC-GGC-GCT 3'

6. Procédé selon la revendication 1, caractérisé en ce que la séquence d'ADN codant pour ledit peptide signal a la formule :

   5' ATG-AAA-TCC-ACG-CTG-CTT-CTC-TTA-TTT

   CTG-CTC-CTG-TGC-CTG-CCC-TCT-TGG-AAC

   GCC-GGC-GCT      -3'

7. Procédé selon la revendication 1, caractérisé en ce que lesdites bactéries appartiennent à l'espèce Escherichia coli.

8. Procédé selon la revendication 7, caractérisé en ce que lesdites bactéries portent une mutation cya par délétion et une mutation crp par délétion.

9. Procédé selon l'une des revendications 7 ou 8, caractérisé en ce que le polypeptide est une forme naturelle ou un variant de l'hirudine.

10. Procédé selon la revendication 9, caractérisé en ce que le variant de l'hirudine est le HV2 (Lys$^{47}$).

**11.** Procédé selon l'une des revendications 7 ou 8, caractérisé en ce que le polypeptide est l'hormone de croissance humaine.

**Claims**

**Claims for the following Contracting States : AT, BE, CH, LI, DE, FR, GB, GR, IT, LU, NL, SE**

**1.** Signal peptide of the formula

    M X K S T L L L L F L L L C L P S W N A G A

where

| | |
|---|---|
| A = Alanine | M = Methionine |
| C = Cysteine | N = Asparagine |
| F = Phenylalanine | P = Proline |
| G = Glycine | S = Serine |
| K = Lysine | T = Threonine |
| L = Leucine | W = Tryptophan |

and X represents a direct bond between M and K, an amino acid selected from the group comprising the 20 amino acids of the genetic code, or a peptide containing 2, 3 or 4 amino acids selected, each independently of the other, from the group comprising the 20 amino acids of the genetic code.

**2.** Signal peptide according to claim 1, characterized in that X represents a direct bond between M and K, or all or part of the peptide of the sequence APSG.

**3.** Signal peptide according to claim 2, characterized in that X represents a direct bond.

**4.** Signal peptide according to claim 2, characterized in that X represents the peptide of the sequence APSG.

**5.** DNA sequence coding for a signal peptide according to any one of claims 1 to 4.

**6.** Sequence according to claim 5, characterized in that it has the formula

        5'      ATG-GCT-CCA-TCT-GGC-AAA-TCC-ACG-CTG

                CTT-CTC-TTA-TTT-CTG-CTC-CTG-TGC-CTG

                CCC-TCT-TGG-AAC-GCC-GGC-GCT                3'

**7.** Sequence according to claim 5, characterized in that it has the formula

        5'      ATG-AAA-TCC-ACG-CTG-CTT-CTC-TTA-TTT-CTG

                CTC-CTG-TGC-CTG-CCC-TCT-TGG-AAC-GCC-GGC

                GCT                                        3'

**8.** Expression vector carrying a DNA sequence coding for a precursor of a polypeptide, characterized in that the portion of this sequence which codes for the signal peptide is a sequence according to any one of claims 5 to 7.

**9.** Gram-negative bacteria characterized in that they are transformed by a vector according to claim 8.

**10.** Bacteria according to claim 9, characterized in that they belong to the species Escherichia coli.

**11.** Bacteria according to claim 10, characterized in that they carry a cya mutation by deletion and a crp mutation by deletion.

**12.** Bacteria according to one of claims 9 and 10, characterized in that the polypeptide is a natural form or a variant of hirudin.

**13.** Bacteria according to claim 12, characterized in that the hirudin variant is (Lys$^{47}$) HV2.

**14.** Bacteria according to one of claims 9 and 10, characterized in that the polypeptide is human growth hormone.

**15.** Process for the periplasmic production of a polypeptide, which consists in cultivating cells of Gram-negative bacteria transformed by an expression vector for a precursor of a polypeptide, in subjecting the cells to an osmotic shock and in separating the recombinant polypeptide from the osmotic shock supernatant, characterized in that the Gram-negative bacteria are bacteria according to one of claims 9 to 14.

**Claims for the following Contracting State : ES**

**1.** Process for the periplasmic production of a polypeptide, characterized in that it consists in cultivating Gram-negative bacteria transformed by an expression vector coding for a precursor of said polypeptide, said precursor comprising a signal peptide of the formula
M X K S T L L L L F L L L C L P S W N A G A

| | |
|---|---|
| A = Alanine | M = Methionine |
| C = Cysteine | N = Asparagine |
| F = Phenylalanine | P = Proline |
| G = Glycine | S = Serine |
| K = Lysine | T = Threonine |
| L = Leucine | W = Tryptophan |

and X represents a direct bond between M and K, an amino acid selected from the group comprising the 20 amino acids of the genetic code, or a peptide containing 2, 3 or 4 amino acids selected, each independently of the other, from the group comprising the 20 amino acids of the genetic code, in subjecting the cells to an osmotic shock and in separating the recombinant polypeptide from the osmotic shock supernatant.

**2.** Process according to claim 1, characterized in that X represents a direct bond between M and K, or all or part of the peptide of the sequence APSG.

**3.** Process according to claim 2, characterized in that X represents a direct bond.

**4.** Process according to claim 2, characterized in that X represents the peptide of the sequence APSG.

**5.** Process according to claim 1, characterized in that the DNA sequence coding for said signal peptide has the formula

```
5'      ATG-GCT-CCA-TCT-GGC-AAA-TCC-ACG-CTG
        CTT-CTC-TTA-TTT-CTG-CTC-CTG-TGC-CTG
        CCC-TCT-TGG-AAC-GCC-GGC-GCT              3'
```

**6.** Process according to claim 1, characterized in that the DNA sequence coding for said signal peptide has the formula

```
5'     ATG-AAA-TCC-ACG-CTG-CTT-CTC-TTA-TTT-CTG
       CTC-CTG-TGC-CTG-CCC-TCT-TGG-AAC-GCC-GGC
       GCT                                      3'
```

7. Process according to claim 1, characterized in that said bacteria belong to the species <u>Escherichia coli</u>.

8. Process according to claim 7, characterized in that said bacteria carry a cya mutation by deletion and a crp mutation by deletion.

9. Process according to one of claims 7 or 8, characterized in that the polypeptide is a natural form or a variant of hirudin.

10. Process according to claim 9, characterized in that the hirudin variant is $(Lys^{47})$ HV2.

11. Process according to one of claims 7 or 8, characterized in that the polypeptide is human growth hormone.

**Patentansprüche**
**Patentansprüche für folgende Vertragsstaaten : AT, BE, CH, LI, DE, FR, GB, GR, IT, LU, NL, SE**

1. Signalpeptid der Formel
   MXKSTLLLLFLLLCLPSWNAGA,
   in der bedeuten:

| | |
|---|---|
| A = Alanin | M = Methionin |
| C = Cystein | N = Asparagin |
| F = Phenylalanin | P = Prolin |
| G = Glycin | S = Serin |
| K = Lysin | T = Threonin |
| L = Leucin | W = Tryptophan |

und X eine direkte Bindung zwischen M und K, eine Aminosäure, die aus der Gruppe der 20 Aminosäuren des genetischen Codes ausgewählt ist, oder ein Peptid, das aus 2, 3 oder 4 Aminosäuren besteht, von denen jede unabhängig von den anderen aus der Gruppe der 20 Aminosäuren des genetischen Codes ausgewählt ist.

2. Signalpeptid nach Anspruch 1, dadurch gekennzeichnet, daß X eine direkte Bindung zwischen M und K oder das ganze Peptid der Sequenz APSG oder einen Teil des Peptids der Sequenz APSG darstellt.

3. Signalpeptid nach Anspruch 2, dadurch gekennzeichnet, daß X eine direkte Bindung ist.

4. Signalpeptid nach Anspruch 2, dadurch gekennzeichnet, daß X das Peptid der Sequenz APSG ist.

5. DNA-Sequenz, die das Signalpeptid nach einem der Ansprüche 1 bis 4 codiert.

6. Sequenz nach Anspruch 5, dadurch gekennzeichnet, daß sie die Formel

```
5'-ATG-GCT-CCA-TCT-GGC-AAA-TCC-ACG-CTG
   CTT-CTC-TTA-TTT-CTG-CTC-CTG-TGC-CTG
   CCC-TCT-TGG-AAC-GCC-GGC-GCT-3'
```

hat.

7. Sequenz nach Anspruch 5, dadurch gekennzeichnet, daß sie die Formel

$$5'-ATG-AAA-TCC-ACG-CTG-CTT-CTC-TTA-TTT$$
$$CTG-CTC-CTG-TGC-CTG-CCC-TCT-TGG-AAC$$
$$GCC-GGC-GCT-3'$$

hat.

8. Expressionsvektor, der eine DNA-Sequenz aufweist, die eine Vorläuferverbindung eines Polypeptids codiert, dadurch gekennzeichnet, daß der Teil dieser Sequenz, der das Signalpeptid codiert, eine Sequenz nach einem der Ansprüche 5 bis 7 ist.

9. Gramnegative Bakterien, dadurch gekennzeichnet, daß sie durch einen Vektor nach Anspruch 8 transformiert sind.

10. Bakterien nach Anspruch 9, dadurch gekennzeichnet, daß sie zu der Art Escherichia coli gehören.

11. Bakterien nach Anspruch 10, dadurch gekennzeichnet, daß sie eine cya-Mutation durch Deletion und eine crp-Mutation durch Deletion aufweisen.

12. Bakterien nach einem der Ansprüche 9 und 10, dadurch gekennzeichnet, daß das Polypeptid eine natürliche oder eine abgewandelte Form des Hirudins ist.

13. Bakterien nach Anspruch 12, dadurch gekennzeichnet, daß die abgewandelte Form des Hirudins HV2 (Lys$^{47}$) ist.

14. Bakterien nach einem der Ansprüche 9 und 10, dadurch gekennzeichnet, daß das Polypeptid das Wachstumshormon des Menschen ist.

15. Verfahren zur periplasmatischen Herstellung eines Polypeptids, das folgende Schritte umfaßt:
   - Kultivierung gramnegativer Bakterienzellen, die durch einen Expressionsvektor einer Vorläuferverbindung eines Polypeptids transformiert sind,
   - Anwendung eines osmotischen Schocks auf die Zellen,
   - Abtrennung des rekombinanten Polypeptids aus dem Überstand des osmotischen Schocks, dadurch gekennzeichnet, daß die gramnegativen Bakterien Bakterien nach einem der Ansprüche 9 bis 14 sind.

**Patentansprüche für folgenden Vertragsstaat : ES**

1. Verfahren zur periplasmatischen Herstellung eines Polypeptids, gekennzeichnet durch folgende Schritte:
   - Kultivierung gramnegativer Bakterien, die durch einen Expressionsvektor transformiert sind, der eine Vorläuferverbindung des Polypeptids codiert, wobei die Vorläuferverbindung ein Signalpeptid der Formel
      MXKSTLLLLFLLLCLPSWNAGA,
      enthält, in der bedeuten:

| | |
|---|---|
| A = Alanin | M = Methionin |
| C = Cystein | N = Asparagin |
| F = Phenylalanin | P = Prolin |
| G = Glycin | S = Serin |
| K = Lysin | T = Threonin |
| L = Leucin | W = Tryptophan |

und X eine direkte Bindung zwischen M und K, eine Aminosäure, die aus der Gruppe der 20 Aminosäuren des genetischen Codes ausgewählt ist, oder ein Peptid, das aus 2, 3 oder 4 Aminosäuren besteht, von denen jede unabhängig von den anderen aus der Gruppe der 20 Aminosäuren des genetischen Codes ausgewählt ist,

- Anwendung eines osmotischen Schocks auf die Zellen und
- Abtrennung des rekombinanten Polypeptids aus dem Überstand des osmotischen Schocks.

2. Verfahren nach Anspruch 1, dadurch gekennzeichnet, daß X eine direkte Bindung zwischen M und K oder das ganze Peptid oder einen Teil des Peptids der Sequenz APSG darstellt.

3. Verfahren nach Anspruch 2, dadurch gekennzeichnet, daß X eine direkte Bindung ist.

4. Verfahren nach Anspruch 2, dadurch gekennzeichnet, daß X das Peptid der Sequenz APSG ist.

5. Verfahren nach Anspruch 1, dadurch gekennzeichnet, daß die DNA-Sequenz, die das Signalpeptid codiert, die Formel

$$5'-ATG-GCT-CCA-TCT-GGC-AAA-TCC-ACG-CTG$$
$$CTT-CTC-TTA-TTT-CTG-CTC-CTG-TGC-CTG$$
$$CCC-TCT-TGG-AAC-GCC-GGC-GCT-3'$$

hat.

6. Verfahren nach Anspruch 1, dadurch gekennzeichnet, daß die DNA-Sequenz, die das Signalpeptid codiert, die Formel

$$5'-ATG-AAA-TCC-ACG-CTG-CTT-CTC-TTA-TTT$$
$$CTG-CTC-CTG-TGC-CTG-CCC-TCT-TGG-AAC$$
$$GCC-GGC-GCT-3'$$

hat.

7. Verfahren nach Anspruch 1, dadurch gekennzeichnet, daß die Bakterien zu der Art Escherichia coli gehören.

8. Verfahren nach Anspruch 7, dadurch gekennzeichnet, daß die Bakterien eine cya-Mutation durch Deletion und eine crp-Mutation durch Deletion aufweisen.

9. Verfahren nach einem der Ansprüche 7 und 8, dadurch gekennzeichnet, daß das Polypeptid eine natürliche oder eine abgewandelte Form des Hirudins ist.

10. Verfahren nach Anspruch 9, dadurch gekennzeichnet, daß die abgewandelte Form des Hirudins HV2 (Lys$^{47}$) ist.

11. Verfahren nach einem der Ansprüche 7 und 8, dadurch gekennzeichnet, daß das Polypeptid das Wachstumshormon des Menschen ist.

# FIG.1

*Plasmide p 163,1*

FIG. 2

*Plasmide p 160,1*

FIG.3

*Plasmide p 373,2*

# FIG. 4

Plasmide p 400,18

FIG.5

Plasmide p 460